# EUROPEAN PATENT APPLICATION

(11) **EP 1 207 197 A1**
(43) Date of publication of application: **22.05.2002**
(21) Application number: 00951534.7
(22) Date of filing: 28.07.2000
(51) Int. Cl.: C12N 9/24, A61K 38/47, A01N 63/02

(54) **ANTIFUNGIC COMPOSITIONS AND METHOD FOR CONTROLLING FUNGI**

(30) Priority: 31.07.1999 ES 9901746
(71) Applicant: NEWBIOTECHNIC, S.A., 41092 Sevilla (ES); Universidad De Sevilla, Vicerrectorado De Investigacion, 41013 Sevilla (ES)
(72) Inventor: REY BARRERA, Manuel, E-41092 Sevilla (ES); SOLER CASTELLS, Andres, E-41092 Sevilla (ES); AIT LAHSEN, Hassane, E-41092 Sevilla (ES); DE LA CRUZ DIAZ, Jesus, E-41092 Sevilla (ES); MONTE VAZQUEZ, Enrique, E-41092 Sevilla (ES); LLOBELL GONZALEZ, Antonio, E-41092 Sevilla (ES)
(86) International application number: ES0000292
(87) International publication number: WO0109294

(57) **Abstract**

The invention relates to the use as an anti-fungal agent of a protein having at least, α-(1,3)-glucanase enzymatic activity, to anti-fungal compositions comprising said protein and to methods for controlling, preventing and/or treating infections caused by pathogenic fungi of plants, animals, including man, contaminants of cultures and food contaminants.

## Description

### FIELD OF THE INVENTION

The invention relates to the use of a protein as an anti-fungal agent. Said protein has at least α-(1,3)-glucanase enzymatic activity. The invention also relates to anti-fungal compositions containing said protein and to methods for controlling, preventing and/or treating infections caused by harmful fungi.

### BACKGROUND OF THE INVENTION

Numerous fungal species can act as pathogens in plants that are of great economic importance. Such species cause large losses in yield and quality. To prevent and reduce diseases caused by phytopathogenic fungi, diverse compounds with anti-fungal activity have been identified, both natural and synthetic.

Moreover, in certain situations opportunistic fungal infections occur caused by different species of fungi, such as *Candida* sp., *Aspergillus* sp., etc., which can cause serious pathological conditions, especially in immuno-compromised patients, for example, oropharingeal candidiasis in AIDS patients. Despite the fact that diverse fungicides are used in human or animal therapy, based on polyenes or azoles, the search continues for effective anti-fungal agents to treat said infections.

A common characteristic of fungi, in particular, filamentous fungi and yeast-like fungi, is the existence of cell walls. The chemical structure of the fungal cell wall is rather complex and has not been well studied. In general, it is known that the cell wall is formed of a chitin or cellulose skeleton and of β-1,3-glucans along with other minor polymers, such as β-1,6-glucans or α-(1,3)-glucans, but which seem to have a critical function in the maintenance of the wall structure.

A specific way of destroying the cell wall of a fungus would be by means of the use of hydrolytic enzymes of polymers constituting the cell wall. Although numerous hydrolytic enzymes of such polymers are known, only a minority of them have the capacity to destroy an intact fungal cell wall (lytic capacity). In some cases, the lytic capacity resides in the capacity of the hydrolytic enzyme to bind to the cell wall of the fungus to be destroyed. Such a capacity depends on the binding domain to the wall of the hydrolytic enzyme that is independent of the domain responsible for enzymatic activity. Therefore, in the search for selective anti-fungal agents, said hydrolytic enzymes with capacity for binding to fungal cell walls would be very promising candidates.

As the chitin and the β-1,3-glucan are the main structural components of the cell walls of the fungi (except in the case of the *Oomycetes,* which contain β-glucans and cellulose), it has been proposed that the chitinases and the β-1,3-glucanases are the key enzymes in the lysis of the cell walls of phytopathogenic fungi. However, it seems that other enzymes that degrade the cell walls, including those that hydrolyse the minor polymers (β-1,6-glucans, α-1,3-glucans, etc.) may also be implicated in the efficient and complete degradation of the cell walls of the mycelium and of the conidia of phytopathogenic fungi.

The polysaccharides that contain α-glycoside bonds seem to play an important role in the architecture and composition of the cell wall of the fungi. The glucans known as alkali soluble glucans (S-glucan) include those ranging from polymers that contain only α-(1,3)-glycoside bonds to polymers that contain α-(1,3)-glycoside and α-(1,4)-glycoside bonds that alternate regularly [Bartnicki-García, Annu. Rev. Microbiol., 1968, 22, 87-109; Bacon et al., Biochim, Biophys. Acta, 1968, 158, 313-315]. The S-glucan represents the main polysaccharide of the matrix of many fungi, and reaches approximately 25% of the dry weight of the cell wall of *Aspergillus nidulans.* Despite the importance of S-glucan, little is known of the existence of enzymatic activities capable of hydrolysing said polymer.

The α-(1,3)-glucanases [glucan (1,3)-α-glycosidase, EC 3.2.1.84] are enzymes able to hydrolyse α-(1,3)-glycoside bonds. There is not much information available on these enzymes. The Japanese patent application JP 04058889 describes an enzyme with α-(1,3)-glucanase activity from *Trichoderma harzianum*, which degrades mutane (mutanase) an extracellular polymer of α-(1,3)-glucan produced by *Streptococcus* sp. that colonises the teeth. Patent application WO 98/00528 describes a recombinant mutanase derived from *T*. *harzianum* and its use in products for oral hygiene.

### SUMMARY OF THE INVENTION

The invention addresses the problem of providing agents with anti-fungal activity.

The solution provided by this invention is based on the fact that the inventors have identified a protein that possesses, at least α-(1,3)-glucanase activity, and also surprisingly has anti-fungal activity, and so it is of use for controlling fungi, in particular, fungi containing polymers in their cell wall having α-(1,3)-glycoside bonds, for example, S-glucan.

The anti-fungal activity of said protein has been shown by means of assays of the inhibition of the germination of spores and/or the inhibition of growth of mycelium of fungi and assays of binding to the cell walls of the fungi.

Therefore, an object of this invention consists in the use as an anti-fungal agent of a protein having at least α-(1,3)-glucanase activity.

An additional object of this invention consists of an anti-fungal composition comprising said protein useful as an anti-fungal agent.

Another additional object of this invention consists of a pharmaceutical composition containing said protein useful as an anti-fungal agent.

Another additional object of this invention consists of a method for inhibiting the germination of spores and/or the growth of mycelium of fungi, which comprises bringing said fungi into contact with said anti-fungal composition.

Another additional object of this invention consists of a method for controlling phytopathogenic fungi, which comprises bringing said fungi into contact with said anti-fungal composition.

Another additional object of this invention consists of a method for preventing and/or treating infections caused by pathogenic fungi in animals, including human beings, which comprises administering the animal or patient in need thereof with a therapeutically effective amount of said protein useful as an anti-fungal agent or of said pharmaceutical composition.

Another additional object of this invention consists of using said protein useful as an anti-fungal agent in the production of a medicament for the treatment and/or prevention of diseases caused by fungi.

### BRIEF DESCRIPTION OF THE DRAWINGS

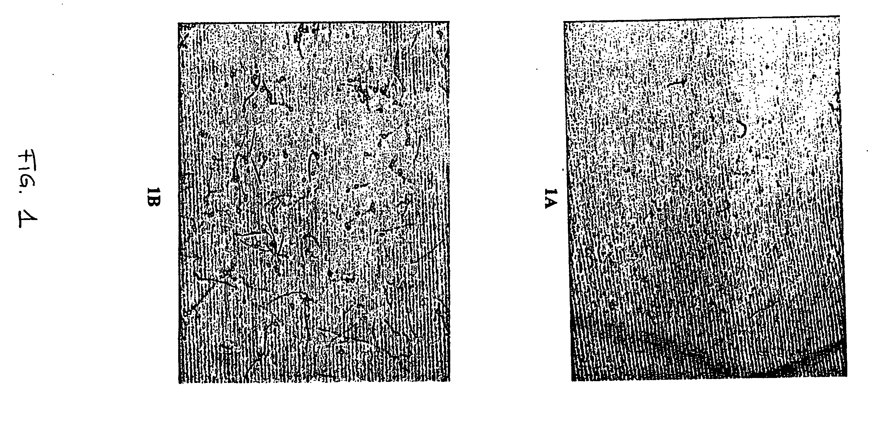

Figure 1 shows an image taken from an optical microscope where the anti-fungal effect can be seen, determined by the inhibition of growth of the phytopathogenic fungus *Penicillium digitatum,* produced by the anti-fungal protein AGN13.1 from *T. harzianum* [Figure 1A] and the comparison with a negative control [Figure 1B],

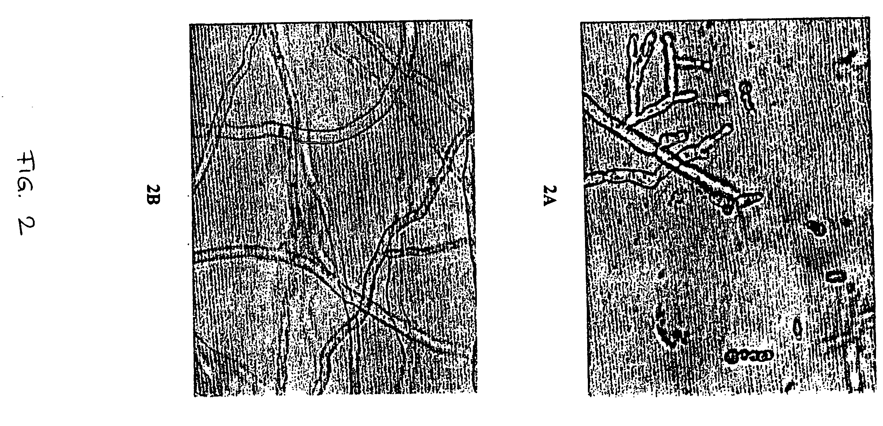

Figure 2 shows an image taken from an optical microscope in which the anti-fungal effect can be seen, determined by the morphological alterations of the mycelium of the phytopathogenic fungus P. *digitatum,* produced by the anti-fungal protein AGN13.1 [Figure 2A] and the comparison with a negative control [Figure 2B].

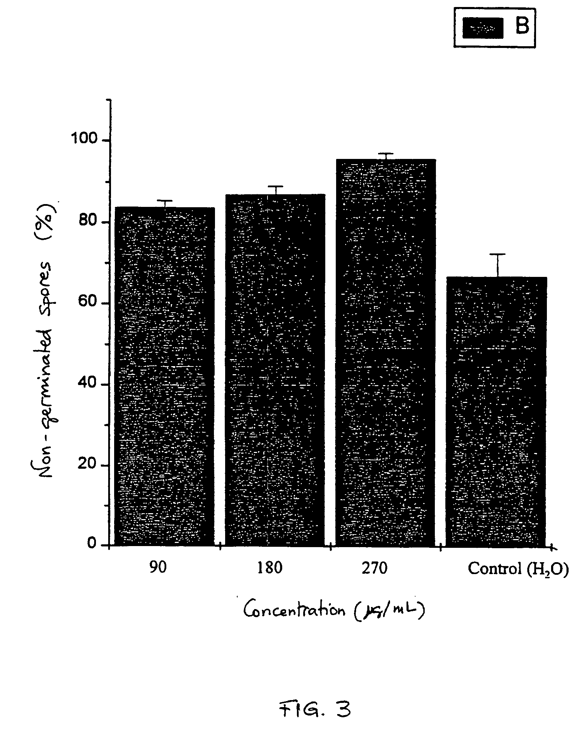

Figure 3 is a bar diagram that shows the anti-fungal effect of the enzyme AGN13.1 from *T. harzianum* on the germination of spores of *Aspergillus niger* CECT 2574 determined after 12 hours [Example 11].

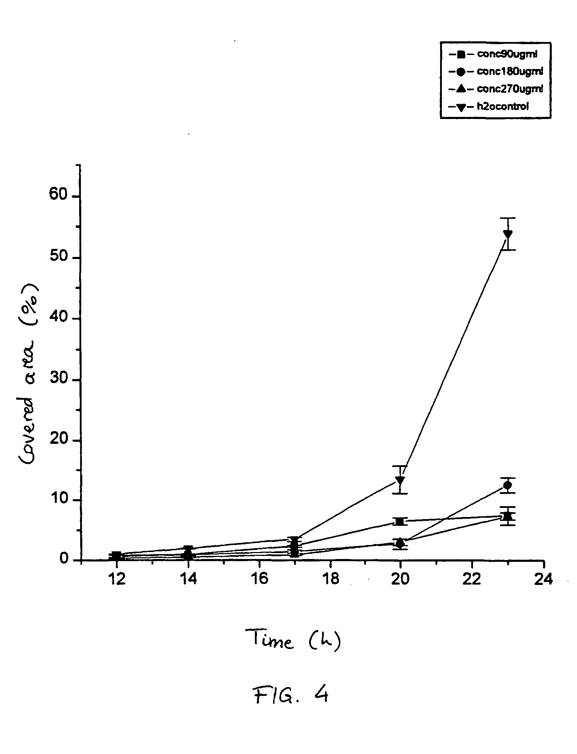

Figure 4 is a plot that shows the effect of the enzyme AGN13.1 from *T*. *harzianum* on the growth of *A*. *niger* CECT 2574 [Example 11].

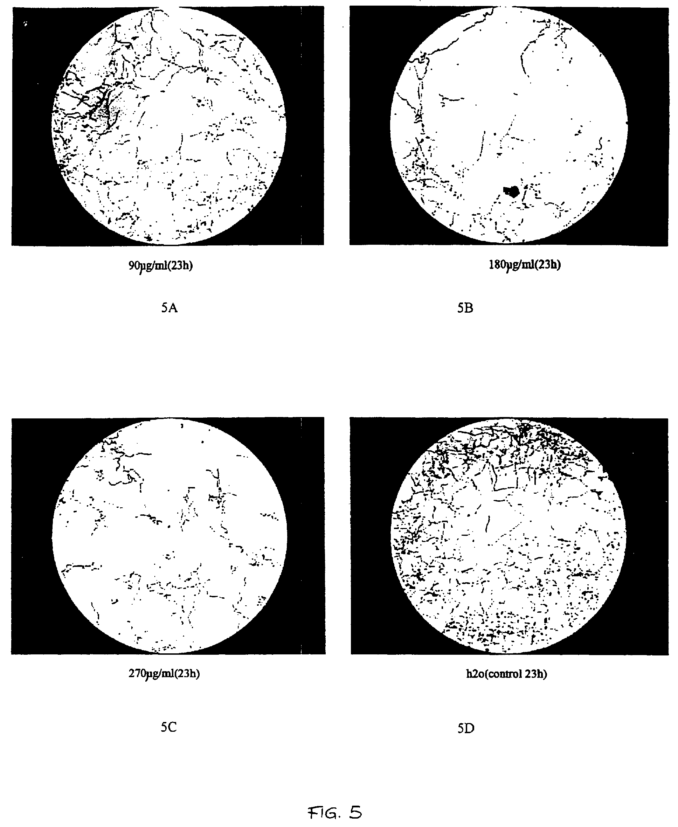

In Figure 5, there is shown an image taken from an optical microscope in which the anti-fungal effect is seen, determined by the morphological alterations of the mycelium of A. *niger* CECT 2574, after 23 hours, produced by the anti-fungal protein AGN13.1 from *T. harzianum* at different concentrations: 90 µg/ml [Figure 5A]_{,} 180 µg/ml [Figure 5B] and 270 µg/ml [Figure 5C] and the comparison with a negative control based on water [Figure 5D].

### DETAILED DESCRIPTION OF THE INVENTION

The invention relates to the use of a protein having at least α-(1,3)-glucanase activity as an anti-fungal agent, hereinafter the anti-fungal protein.

### ANTI-FUNGAL PROTEIN

The anti-fungal protein is a protein that has at least α-(1,3)-glucanase activity and the capacity to bind to a cell wall of a fungus, in particular, a fungus that contains in its cell walls a fungus that contains α-(1,3)-glycoside bonds, and to inhibit the germination of spores and/or the growth of the mycelium of a fungus, in particular, a fungus containing in its cell wall a polymer that contains α-(1,3)-glycoside bonds.

In a particular embodiment of the invention, said anti-fungal protein has a sequence of amino acids selected from:
a) The sequence of amino acids shown in SEQ. ID No. 1, and
b) A sequence of amino acids substantially homologous and functionally equivalent to the sequence of amino acids shown in SEQ. ID No. 1.

In the sense used in this description, the expression "substantially homologous" means that the sequences of amino acids in question have a degree of identity, at an amino acid level of at least 70%, preferably of at least 85% and more preferably of at least 95%.

Similarly, in the sense used in this description, the expression "functionally equivalent" means that the protein in question has α-(1,3)-glucanase activity and anti-fungal activity, determined in the latter of these cases by means of the capacity to bind to a cell wall of a fungus, in particular, a fungus that contains in its cell wall a polymer having α-(1,3)-glycoside bonds, and to inhibit the germination of spores and/or the growth of the mycelium of a fungus, in particular, of a fungus that contains in its cell wall a polymer having α-(1,3)-glycoside bonds.

The α-(1,3)-glucanase activity can be determined by means of the assays described in Example 2; the binding capacity of the anti-fungal protein to components of the cell walls of fungi can be determined following the protocol described in Example 9; the capacity of binding of the anti-fungal protein to cell walls of fungi can be determined by means of the protocol described in Example 11; and the anti-fungal activity of the anti-fungal protein can be checked by means of the protocol described in Example 10.

In a particular embodiment of the invention, said anti-fungal protein is a α-(1,3)-glucanase from *T. harzianum,* for example, α-(1,3)-glucanase identified as AGN13.1 and with the sequence of amino acids shown in SEQ. ID. No.: 1.

Said protein AGN13.1 presents the following characteristics:
It is a monomeric enzyme that degrades α-(1,3)-glucan,
it has an apparent molecular weight of approximately 72 kDa (determined by SDS-PAGE) and of approximately 67 kDa (determined by gel filtration chromatography),
it has an isoelectric point of approximately 7 (determined by the isoelectric focussing) and of approximately 7.6 (determined by chromatofocussing),
its mechanism of action is of the exo type
its hydrolytic activity is specific to the α-(1,3)-glycoside bonds,
it is induced in conditions that simulate the antagonistic response of *T*. *harzianum* (determined by means of Northern Blot and Western Blot analysis), and
it is able to bind to cell walls of some fungi, hydrolyse them and inhibit the germination of spores and the growth of mycelium of said fungi.

The anti-fungal protein can be obtained from a producing organism, by means of a procedure that comprises cultivating said microorganism under conditions appropriate for the expression of said protein and recovering it. In a particular embodiment of this invention, the producing organism is *T. harzianum* [Example 1].

The isolation and purification of the anti-fungal protein can be carried out by means of its adsorption to cell walls of fungi and release by digestion of these cell walls, by means of a process comprising precipitation of the protein with ammonium sulphate, the adsorption-digestion at cell walls of fungi or at components of said walls, and the separation by gel filtration chromatography of the concentrated fractions presenting anti-fungal activity [Example 3]. This method allows isolation of proteins with affinity for the structure of the components of the cell walls of fungi.

The anti-fungal protein according to this invention is able to hydrolyse polymers having α-(1,3)-glycoside bonds, for example, polymers of α-(1,3)-glucan. The cell walls of some fungi contain this type of polymer, among others (chitin, β-(1,3)-glucans, β-(1,6)-glucans, etc), and so said anti-fungal protein can be used to control fungi that contain in their cell walls polymers having α-(1,3)-glycoside bonds. In a particular embodiment, it has been observed that the anti-fungal protein according to the invention is able to degrade S-glucan, and so it can be used for inhibiting the germination of spores and/or the growth of mycelium of fungi that contain S-glucan in their cell walls, and, therefore, for controlling fungi that contain said polymer in their cell wall. The term "fungus", as used in this description, includes yeasts. In a particular embodiment, said fungi are harmful fungi that contain in their cell wall a polymer that contains α-(1,3)-glycoside bonds, for example, S-glucan. In the sense used in this description, the term "harmful fungi" includes pathogenic fungi of plants (including the fungi that contaminate fruit before and after harvesting), pathogenic fungi of animals (including pathogens of humans), fungi that contaminate food and, in general, any fungi that causes economic losses in any industrial, agricultural or livestock sectors. By way of example, the fungi susceptible to attack by the anti-fungal protein according to this invention are fungi that contain in their cell wall the target polymer (polymers that contain the α-(1,3)-glycoside bonds) and which spread in the Fungi Kingdom, from yeasts through to filamentous fungi.

Among the phytopathogenic fungi susceptible to control by the anti-fungal protein according to this invention are, by way of example which is not limiting, the Ascomycetes and Basidiomycetes, in their sexual and asexual phases, for example, the microorganisms causing diseases such as rust, smut, chancres, root mycosis, foliar mycosis, vascular mycosis, post-harvest mycosis, etc.

Among the pathogenic fungi in animals, including human beings, which are susceptible to control by the anti-fungal protein according to the invention, by way of example which is not limiting, are the fungi implicated in diseases such as pityriasis *(Malassezia furfur),* black calculus *(Piedraia hortae),* white calculus *(Trichosporon beigelii),* dermatomycosis *(Microsporum, Trichophyton, Epidermophyton),* sporotrichosis *(Sporothrix schenkii),* chromoblastomycosis (yeasts and hyphomycetes dermatiaceous), pheohyphomycosis *(Fonsecaea pedrosei, F. compacta, Cladosporium carrionii, Phialophora verrucosa),* mycotic mycetomas *(Madurella grisea, Pseudalesheria boydii),* histoplasmosis *(Histoplasma capsulatum* = *Ajellomyces capsulatus),* blastomycosis *(Blastomyces dermatiditis),* paracoccidiosis *(Paracoccidiodes brasilieniss),* aspergillosis *(Aspergillus* sp., especially, *A. nidulans, A. niger, A. fumigatus* and *A. flavus),* opportunistic mycosis *(Candida* sp., *Pneumocytis carinii),* cygomycosis *(Rhizopus* sp., *Absidia* sp., *Mucor* sp.) and cryptococcosis *(Filobasidiella neoformans = Cryptococcus neoformas).*

### ANTI-FUNGAL COMPOSITION

The invention provides an anti-fungal composition that comprises, at least, an anti-fungal protein according to the invention, along with an inert vehicle, such as a diluent, a solvent and a surfactant.

In the sense used in this specification, the term "inert" is used to indicate that said vehicle does not have significant anti-fungal activity.

Said anti-fungal composition may contain in addition other natural fungicides, recombinant or synthetic, that enhance the action of the anti-fungal protein according to the invention.

This anti-fungal composition may be used for the de-infestation of surfaces, equipment, apparatus of any field of technology, for example, industrial, agricultural or livestock, by means of its application to said surfaces, equipment or apparatus.

### ANTI-FUNGAL COMPOSITION FOR CONTROLLING PHYTOPATHOGENIC FUNGI

An important field where the object of the present invention finds application is in the agricultural field. In this sense, the invention provides a composition for controlling phytopathogenic fungi that comprises an effective anti-fungal quantity of said anti-fungal protein according to the invention, along with, optionally, one or more agriculturally acceptable vehicles. This composition has good anti-fungal activity against a broad group of phytopathogenic fungi, in particular, against phytopathogenic fungi that contain in their cell wall polymers having α-(1,3)-glycoside bonds.

In the sense used in this specification, the term "to control" includes the inhibition, reduction or paralysing the germination of spores and/or the growth of mycelium of fungi that may result in the elimination of said fungi or in the reduction in the damage caused thereby.

In the sense used in this specification, the expression "effective anti-fungal quantity" refers to the amount of anti-fungal protein able to inhibit the germination of spores and/or the growth of the mycelium of the target fungus when said protein is applied to the plant, or to the medium that surrounds it or when it is expressed in a transgenic plant.

In the sense used in this specification, the expression "agriculturally acceptable vehicle" refers to those substances, or combination of substances, known in the agricultural field, and includes adjuvants, solids or liquids, diluents, solvents, surfactants, etc.

In a particular embodiment, said composition comprises, in addition to said anti-fungal protein, one or more proteins, with same or different enzymatic activities, for example, different enzymatic activities required for the modification or degradation of cell walls. By way of example, said composition may include one or more lytic enzymes able to modify or degrade cell walls of fungi, for example, enzymes with cellular lytic activity, mananolytic activity, chitinolytic activity or proteolytic activity, such as cellulases, α-(1,3)-glucanases, β-(1,6)-glucanases, (β-(1,3)-glucanases, mananases, endo- and exo- chitinases, chitosanases, proteases, α-or β-manosidases, etc. These enzymes may originate from any producing organism thereof.

In another particular embodiment, the composition for controlling phytopathogenic fungi comprises, in addition, one or more chemical fungicides. As a chemical fungicide any of the chemical fungicides normally used can be used, preferably a chemical fungicide selected from the group formed by the fungicides that affect the membrane, the fungicides that affect the synthesis of the cell wall and mixtures thereof.

The composition for controlling phytopathogenic fungi provided by this invention contains between 0.01% and 100% by weight of said anti-fungal protein, can be prepared by conventional methods and can be presented in a liquid or a solid form, for example, in the form of a granulate. In addition, said composition may contain additives, for example, preservatives and stabilizers that prolong its conservation and stability.

### METHOD FOR CONTROLLING PHYTOPATHOGENIC FUNGI

In another aspect, the invention provides a method for controlling a phytopathogenic fungus in a plant, in particular, a fungus that contains in its cell wall a polymer having α-(1,3)-glycoside bonds, for example, S-glucan, which comprises the stage of applying to said plant, or to the surrounding environment, an effective quantity of said composition for controlling said phytopathogenic fungus.

In a particular embodiment, said method comprises the application of said composition to the parts of the plant above ground in order to prevent and/or treat a fungal infection caused by a phytopathogenic fungus.

In another aspect, the invention provides a method for controlling phytopathogenic fungi in fruit, which comprises the stage of applying to a fruit an effective quantity of said composition for controlling phytopathogenic fungi. In a particular embodiment of said method, the application of said composition to the fruit is carried out before its harvest (pre-harvest) while in an alternative embodiment the application of the composition to the fruit is carried out with the plant already harvested (post-harvest).

The invention also contemplates the possibility of obtaining transgenic plants that express the anti-fungal protein according to the invention both in specific tissues or organs and in any other part of the plant or fruit. In this way, it is possible to obtain plants and/or fruits resistant to phytopathogenic fungi, which are responsible for extensive damage both before and after the harvest. To obtain these transgenic plants, it is possible to use conventional methods known by those skilled in the art, transforming cells of plants with a DNA construct that contains the sequence coding for an anti-fungal protein according to the invention [see section relating to MOLECULAR BIOLOGY]. The expression of said anti-fungal protein may be regulated by a constitutive or inducible promoter in plants with the aim that said anti-fungal protein is expressed in the moment and place appropriate such that an effective protection is afforded against the phytopathogenic fungus to be controlled.

### PHARMACEUTICAL COMPOSITION FOR THE PREVENTION AND/OR TREATMENT OF FUNGI PATHOGENIC TO ANIMALS

Another important field where the object of this invention can be applied is that of Health, both of humans and animals. In this sense, the invention provides a pharmaceutical composition to prevent and/or treat fungi pathogenic to animals, including pathogens in humans, in particular, fungi that contain in their cell wall polymers having α-(1,3)-glycoside bonds, for example, S-glucan, which comprises an effective quantity of said anti-fungal protein according to the invention, along with, optionally, one or more pharmaceutically acceptable excipients. This composition has a good anti-fungal activity against a broad group of pathogenic fungi in animals.

In the sense used in this description, the expression "prevent and/or treat" includes the inhibition, reduction or paralysis of the germination of the spores and/or of the growth of the mycelium of fungi that may result in the elimination of said fungi or in a reduction of the damage caused thereby.

In the sense used in this specification, the expression "effective anti-fungal quantity" refers to the amount of anti-fungal protein able to inhibit the germination of spores and/or the growth of mycelium of target pathogenic fungus of animals when applied to the animal.

In the sense used in this specification, the expression "pharmaceutically acceptable excipient" refers to those substances, or combination of substances, known in the pharmaceutical field, and includes adjuvants, solids or liquids, solvents, surfactants, etc.

In a particular embodiment, said pharmaceutical composition comprises, in addition to said anti-fungal protein, one or more proteins, with same or different enzymatic activities, for example, different enzymatic activities required for the modification or degradation of cell walls, such as the enzymes mentioned previously regarding the composition for controlling phytopathogenic fungi.

In another particular embodiment, said pharmaceutical composition comprises, in addition, one or more fungicides of those normally used in the treatment of mycosis in animals.

The pharmaceutical composition provided by this invention contains between 0.01% and 99.99% by weight of said anti-fungal protein, and can be presented in any appropriate administration form. In general, said pharmaceutical composition will be administered by topical route, in the form of an ointment or cream. A review of the different pharmaceutical forms of administration of drugs and of their processes for preparation can be found, for example, in the "Tratado de Farmacia Galénica", C. Faulí i Trillo, 1^{st} Edition, 1993, Published by Luzán 5, S.A. de Ediciones.

In another aspect, the invention relates to the use of said anti-fungal protein in the preparation of a medicament for the prevention and/or treatment of the infection of pathogenic fungi in animals, including pathogens in humans.

### METHOD FOR THE PREVENTION AND/OR TREATMENT OF PATHOGENIC FUNGI IN ANIMALS

The invention also provides a method for preventing and/or treating pathogenic fungi in animals, including pathogens in humans, in particular those fungi that have in their cell walls polymers having α-(1,3)-glycoside bonds, for example, S-glucan, which comprises the stage of applying to an animal or a human being, in need of treatment thereof, an effective quantity of said pharmaceutical composition for the prevention and/or treatment of infections of pathogenic fungi in animals.

The live-stock facilities are susceptible to infections by pathogenic fungi in animals that grow and develop in substrates that are in contact with the animals, for example, the straw used for the beds of the animals, or in the stabling conditions of the animals, and so there is a risk that said animals are infected by such pathogens. The anti-fungal protein according to the invention can be used for de-infesting, preventing and/or treating the infection caused by pathogenic fungi of animals in livestock facilities that comprises the stage of applying to said live-stock facilities an effective quantity of said pharmaceutical composition for the prevention and/or treatment of infections by pathogenic fungi of animals.

### ANALYTICAL APPLICATIONS

On occasions, the samples of the assay for analysis, for example, biological samples, of foods, etc., present contaminations by fungi which make the analysis more difficult or impede the analysis of said samples. The invention also provides a solution to said problem that comprises bringing into contact the sample to be analysed and an effective amount of said anti-fungal protein according to the invention, with the aim of controlling the possible fungal contamination.

### MOLECULAR BIOLOGY

Based on the information provided by the anti-fungal protein according to this invention it is possible to identify and isolate the DNA sequence that codes for said protein by means of the use of conventional techniques, for example, by means of the creation of gene libraries of genomic DNA (gDNA) or copy DNA (cDNA) of producing organisms of said protein; the sequence of the amino terminus of said protein and tryptic fragments thereof; the design of oligonucleotides for amplification, by means of the polymerase chain reaction (PCR), a region of the genomic clone of the producing organisms of said protein that serves for obtaining probes for examining said gene libraries; and the analysis and selection of positive clones. All these stages are described in more detail in Examples 12 and 13.

In another aspect, the invention provides a DNA construct that codes for an anti-fungal protein according to the invention, which comprises:
a) a DNA sequence identified as SEQ. ID. No.: 2; or
b) a DNA sequence analogous to the sequence defined in a) which
   i) is substantially homologous with the sequence of DNA defined in a); and/or
   ii) codes for a polypeptide which is substantially homologous with the protein coded for by the DNA sequence defined in a).

In the sense used in this specification, the term "analogous" includes any DNA sequence that codes for an anti-fungal protein according to the invention which has the properties i)-ii) mentioned above. Typically, the analogous DNA sequence can be isolated from an organism that produces the anti-fungal protein and exhibits α-(1,3)-glucanase activity, on the basis of the DNA sequence shown in SEQ. ID. No. 2, or else it is constructed on the basis of the DNA sequence shown in SEQ- ID. No. 2, for example, by means of the introduction of preserving substitutions of nucleotides, in other words, substitutions that do not give rise to another sequence of amino acids of the protein with anti-fungal activity coded by said DNA sequence shown in SEQ. ID. No. 1, but which corresponds to the use of codons of the host organism destined to the production of the protein, or else by means of the introduction of nucleotide substitutions that give rise to a different sequence of amino acids that may give rise to a mutant protein with properties different from those of the native protein. Other examples of possible modifications include the insertion of one or more nucleotides into the sequence, the addition of one or more nucleotides to any of the termini of the sequence, or the deletion of one or more nucleotides at either of the termini of the sequence or in the inner part of the sequence. For example, the analogous DNA sequence may be a sub-sequence of the sequence of DNA shown in any of the sequences shown in SEQ. ID. No. 2.

In general, the analogous DNA sequence is substantially homologous with the DNA sequence that codes for the anti-fungal protein of the invention, for example SEQ. ID. No. 2, in other words, it presents a homology at a nucleotide level of at least 70%, preferably at least 85%, or more preferably at least, 95% with respect to any of the sequences mentioned previously.

In a particular embodiment, said DNA sequence is the sequence identified as SEQ. ID. No. 2, which corresponds to the sequence of nucleotides that code for the complete AGN13.1 from *T*. *harzianum.*

In another aspect, the invention also provides a DNA sequence that codes only for the domain responsible for the binding of said anti-fungal protein according to the invention to the cell wall, selected from the group made up of:
a) a DNA sequence identified as SEQ. ID. No. 3;
b) a DNA sequence which is substantially homologous with the DNA sequence defined in a); and
c) a DNA sequence that codes for a polypeptide which is substantially homologous with the domain responsible for the binding to the cell wall of the anti-fungal protein coded for by the sequence of DNA defined in a).

The SEQ. ID. No. 3 corresponds to the sequence of nucleotides that code for the module responsible for binding to the cell wall that presents the protein AGN13.1.

The DNA sequence that codes for said anti-fungal protein as well as the sequence of DNA that codes only for the domain responsible for the binding to the cell wall of said protein both constitute what is denominated the DNA sequence of the invention.

The DNA sequence of the invention can come from any microorganism that naturally contains it, for example, *T*. *harzianum* or a host organism transformed with said DNA sequence.

Alternatively, the DNA sequence of the invention can be isolated by means of conventional techniques, starting from the DNA of any organism by means of the use of probes or of oligonucleotides prepared from the information on the DNA sequence provided in this specification.

The DNA sequence of the invention, or the construct that contains it, can be inserted into an appropriate vector. Therefore, the invention also relates to a vector, such as an expression vector, that comprises said DNA sequence, or a construct that contains it. The choice of vector will depend on the host cell in which it is subsequently going to be introduced. By way of example, the vector where said DNA sequence is introduced may be a plasmid or a vector which, when introduced into a host cell, is integrated into the genome of said cells and replicates along with the chromosome (or chromosomes) in which it has been integrated.

In the vector provided by this invention, the DNA sequence of the invention should be operatively connected to a promoter and to a terminal sequence. The promoter may be any sequence of DNA that shows transcriptional activity in the chosen host cell and may be derived either from genes that code for homologous proteins or heterologous proteins of the host cell. The procedures used for binding the DNA sequence of the invention to the promoter and the terminal sequence, respectively, and for inserting said construct into a vector are well known by those versed in the art and have been described for example by Sambrook et al. [Molecular Cloning. A Laboratory Manual, Cold Spring Harbor, NY, 1989].

The invention also provides a cell that comprises a sequence of DNA of the invention, or a DNA construct that contains said sequence or said vector mentioned above. The host cells that can be transformed with the DNA sequence of the invention can be prokaryotic cells, or preferably, eukaryotic cells, such as cells of plant tissues or fungal cells, for example, yeast cells or cells of filamentous fungi. The transformation of fungal cells may be carried out by means of conventional techniques, for example, techniques that imply the formation of protoplasts and the transformation thereof followed by the regeneration of the cell wall. The transformation of cells of plant tissue can also be carried out by conventional methods and it may be useful for conferring resistance on the transformed plant against harmful organisms, for example, phytopathogenic fungi.

The invention also provides a method for the production of an anti-fungal protein according to the invention which comprises culturing a suitable host cell that contains the DNA sequence that codes for said anti-fungal protein under conditions that allow the production of the protein and the recovery of the protein from the culture medium.

The medium used for culturing the transformed host cells may be any suitable medium for culturing the host cells in question. The protein expressed can be, advantageously, secreted into the culture medium and can be recovered by means of any conventional procedure for isolation of proteins that comprises the separation of the cells from the culture medium, the precipitation of the proteins and separation by chromatographic means.

The following examples illustrate the present invention and should not be considered as limiting the scope thereof.

### EXAMPLE 1

### Culture of T. harzianum and production of AGN13.1

The culture of *T*. *harzianum* and the production of AGN13.1 may be carried out in several ways using different carbon sources.

### 1.1 Method I: Chitin as a carbon source

*T. harzianum* CECT 2413 was obtained from the *Colección Española de Cultivos Tipo* (Spanish Collection of Culture Types) CECT (Valencia, Spain) and was kept in a PPG culture medium (potato puree + glucose, both at 2%), supplemented with 2% agar.

For the production and purification of AGN13.1, *T. harzianum* 2413 was cultured in two stages following protocols already described by De la Cruz et al. [De la Cruz et al., J. Bacteriol., 1995, Vol 177, No. 7, 1864-1871]. Briefly, in a first stage, spores of *T. harzianum* were inoculated at a final concentration of 10⁸ spores/ml of minimum medium (MM) [KH₂P_{O4}, 15 g/l; (NH₄)₂SO₄, 5 g/l; 1 ml/l of oligoelements FeSO₄·7H₂O, MnSO4.H2O, 1.6 g/l; ZnSO4.7H2O, 1.4 g/l; CoCl₂·6H₂O, 3.7 g/l)] supplemented with glucose at 2% as a carbon source. The final pH of the culture medium was adjusted to 5.5 with 1M KOH. In this pre-induction medium, *T. harzianum* was cultured for 48 hours at 28° C and with orbital shaking at 200 rpm. Subsequently, the mycelium was harvested, washed successively with MgCl₂ and H₂O and transferred to MM supplemented with chitin at 1.5% as source of carbon.

### 1.2 Method II: Other sources of carbon

*T. harzianum* CECT 2413 was kept in a PDA culture medium (potato-dextrose-agar) [Difco]. For the production and purification of AGN13.1, *T*. *harzianum* 2143 was cultured in two stages following protocols already described by De la Cruz et al. [De la Cruz et at., Arch. Microbiol., 1993, 159, 316-322]. Briefly, in a first stage, aliquots of 10⁶ conidia of *T*. *harzianum* 2413 were inoculated in 1L Erlenmayer flasks containing 600 ml of culture broth of potato-dextrose supplemented with Czapek salts [De la Cruz et al., Arch. Microbiol., 1993, 159, 316-322] and they were incubated for 2 days at 26° C and 200 rpm (repression conditions). Next, the mycelia were harvested and washed under sterile conditions, transferred to 1L Erlenmayer flasks containing 600 ml of Czapek minimum medium supplemented with different sources of carbon (glucose, chitin, cell walls of *A*. *niger,* cell walls of *P*. *aurantogriseum,* and cell walls of *P*. *syringae).* The flasks were incubated for 48 hours in the aforementioned conditions (induction conditions) and the levels of extracellular activity of α-(1,3)-glucanase measured by means of the process described in Example 2. In addition, an assay was carried out in conditions of nitrogen deficiency, which corresponds to a reduction by 100 times of the concentration of ammonium chloride in the medium (21 mg/l of ammonium chloride). The results obtained are shown in Table 1.

**Table 1**

| **Effect of the source of carbon on the production of extracellular α-(1,3)-glucanase activity by *T*. *harzianum*** | |
|---|---|
| **Carbon source [% (w/v)]** | **α-(1,3)-glucanase activity (mU/mg of protein)** |
| Glucose (0.1) | 120 |
| Glucose (2) | 120 |
| Chitin(1.5) | 98 |
| Cell walls of *A*. *niger* (0.5) | 380 |
| Cell walls of *P*. *Aurantogriseum* (0.5) | 130 |
| Cell walls of *P*. *Syringae (0.5)* | 320 |
| Nitrogen deficiency | 40 |

As was indicated previously for other enzymes that degrade the cell wall of *T*. *harzianum* [De la Cruz et al., Arch. Microbiol., 1993, 159, 316-322], the α-(1,3)-glucanase activity is repressed at high concentrations of glucose [2% (w/v)] but not in conditions of carbon deficiency [0.1% (w/v)] and is induced in the presence of complete fungal cell walls or their constituents. Therefore, the production of α-(1,3)-glucanase activity [AGN13.1] by *T. harzianum* depends on the available carbon source. From the results presented in Table 1 it follows that cell walls of *A*. *niger* [0.5% (w/v)] can be routinely used for the purification of AGN13.1.

### EXAMPLE 2

### Assay of α-(1,3)-gIucanase activity and determination of the protein concentration

### 2.1 α-(1,3)-glucanase activity

### The activity of α-(1,3)-glucanase can be assayed by different methods.

### 2.1.1 Method A

The activity of α-(1,3)-glucanase can be determined by means of an assay that consists of preparing a reaction mixture containing 0.8 ml of solution of α-(1,3)-glucan at 0.5% (w/v) in potassium acetate buffer 50 mM, pH 5.5, and 0.2 ml of enzymatic solution to be assayed appropriately diluted in the same buffer. The α-(1,3)-glucan was obtained following the previously described protocols [Zonneveld, Biochim. Biophys. Acta, 1971, 258, 541-547]. The reaction mixture is incubated at 37° C for a period of time comprised between 30 minutes and 1 hour, stopping the reaction by heating to 100° C for 7 minutes. Subsequently, the increase in reducing sugars is determined in 0.15 ml of reaction mixture using the Somogyi method [J. Biol. Chem., 1952, 195, 19-23] and Nelson [Methods Enzymol., 1957, 3, 85-86], using glucose at concentrations comprised between 0 and 1 mg/ml as a standard. Enzyme and substrate blanks are included.

One unit of α-(1,3)-glucanase activity is defined as the amount of enzyme that releases 1 µmol of reducing sugar equivalent, expressed as glucose, per minute, under the assay conditions.

The results obtained on assaying the AGN13.1 enzyme of *T*. *harzianum* using this method showed that said enzyme can degrade α-(1,3)-glucan. In addition, the activity of the AGN13.1 enzyme for degrading other polymers present in the fungal cell walls was evaluated using the corresponding assays. Examples of such polymers are α-(1,4)- and α-(1,6)-glucan, chitin, β-(1,3)- and β-(1,6)-glucan, etc. A negative result was obtained in all cases.

### 2.1.2 Method B

The α-(1,3)-glucanase activity can also be determined by means of an assay that consists of preparing a reaction mixture containing 2 ml of a solution of 5 mg/ml of S-glucan [α-(1,3)-glucan] from *A. niger* in potassium acetate buffer 50 mM, pH 5.5, and 50 µl of the enzymatic solution to assay appropriately diluted in the same buffer. The reaction mixture is incubated at 37° C for 1 hour and the reaction stopped by heating at 100°C for 5 minutes. The samples are centrifuged at 5,000 g for 5 minutes. 0.15 ml of supernatant per reaction are collected and the increase in reducing sugars is determined by the method of Somogyi [J. Biol. Chem., 1952, 195, 19-23] and Nelson [Methods Enzymol., 1957, 3, 85-86], using glucose at concentrations comprised between 0 and 1 mg/ml as standards. Enzyme and substrate blanks are included.

One unit of α-(1,3)-glucanase activity is defined as the amount of enzyme that releases 1 µmol of reducing sugar equivalents, expressed as glucose, per minute, under the assay conditions.

The results obtained on assaying the AGN13.1 enzyme from *T*. *harzianum* using this method showed that said enzyme can degrade α-(1,3)-glucan.

### 2.2 Determination of the protein concentration

The protein concentration was measured by means of the method of Bradford [Bradford, Anal. Biochem., 1976, 72, 248-254].

### EXAMPLE 3

### Purification of the AGN13.1 protein from T. harzianum

The purification of the AGN13.1 protein from *T*. *harzianum* was based on its affinity for an insoluble substrate. The purification of said protein can be carried out following various methods based on this characteristic, which comprise precipitation with ammonium sulphate, the adsorption-digestion at an insoluble substrate for which it has affinity and the separation by gel filtration chromatography.

### 3.1 Method A

The purification of the AGN13.1 protein from *T. harzianum* according to this alternative comprises the stages of:

### A) Precipitation with ammonium sulphate

This stage, as with all the others (unless indicated otherwise) was carried out at 4° C. Cultures of *T*. *harzianum* 2143 incubated for 48 hours in MM supplemented with 1.5 % chitin were filtered through Whatman no. 1 paper and centrifuged at 6,000 g for 10 minutes. Next, the supernatant (approximately 200 ml) was precipitated with ammonium sulphate at 80% saturation. After leaving overnight to incubate, the precipitate was recovered centrifuging at 12,000 g for 20 minutes. It was re-suspended in the smallest possible volume of distilled water and dialysed against potassium acetate buffer 50 mM, pH 5.5.

### B) Adsorption-digestion at cell walls

For the adsorption at cell walls, aliquots of 3 ml of supernatant dialysed with 0.6 ml of a solution of 20 mg/ml of purified and lyophilised cell walls *of Penicillium* sp., obtained according to protocols previously described by Tanaka and Phaff [Tanaka and Phaff, J. Bacteriol. 1965, 89, 1570-1580]. The incubation was carried out for 20 minutes with magnetic stirring and subsequent centrifuging at 12,000 g for 10 minutes. The supernatant (the fraction not absorbed by the pustulan) was incubated once more with cell walls (this process is repeated twice). The precipitates obtained after successive adsorptions were washed three times with 3 ml of a solution of 1M NaCl in phosphate-KOH buffer 70 mM, pH 6.0 and, finally, they were resuspended in potassium acetate buffer 50 mM, pH 5.5, with phenylmethylsulphonyl fluoride 1 mM and sodium azide 1 mM. These samples were incubated overnight at 37° C. The clarified fraction from the degradation of the cell walls was centrifuged at 12,000 g for 10 minutes. The supernatant (5-10 ml) was dialysed once more against imidazole-HCl buffer 25 mM, pH 7. In these assay conditions, a majority protein was obtained which is that identified as AGN13.1 in this specification.

### C) Gel filtration

The dialysed solution from stage b) is applied to a column (1.6 x 40 cm) of Sephacryl S-200 HR (Pharmacia) packed in potassium acetate buffer 100 mM, pH 5.5 with KCl 100 mM and eluted with the same buffer at a flow of 7 ml/h. The different fractions obtained were assayed to evaluate the α-(1,3)-glucanase activity. Those fractions were selected that showed highest levels of activity and collected. They were washed in potassium acetate buffer 50 mM, pH 5.5 in a Centricon 10 (Amicon) concentrator. The purified proteins (AGN13.1) were stored at 4° C.

### 3.2 Method B

The process for purification of the AGN13.1 protein from *T*. *harzianum* according to this alternative comprises the following stages:

### A) Precipitation with ammonium sulphate

This stage, as with all the others (unless indicated otherwise) was carried out at 4° C. Cultures of *T*. *harzianum* incubated for 48 hours in Czapek medium supplemented with 0.1% of cell walls of *A*. *Niger* were filtered through Whatman no. 1 paper and centrifuged at 8,000 g for 10 minutes. Next, the supernatant (approximately 1200 ml) was precipitated with ammonium sulphate at 80% saturation. The precipitate was recovered centrifuging at 12,000 g for 20 minutes. It was re-suspended in the smallest possible volume of distilled water and dialysed against potassium acetate buffer 50 mM, pH 5.5. The preparation of crude enzyme (approximately 35 ml) was recovered by centrifuging the dialysed fraction at 12,000 g for 20 minutes.

### B) Adsorption-digestion at S-glucan

Aliquots of 3 ml of crude enzyme were absorbed at S-glucan (5 mg/ml) for 2 hours with magnetic stirring. Sedimentation was performed by centrifuging at 12,000 g for 10 minutes. The fractions adsorbed were washed three times with potassium acetate buffer 50 mM, pH 5.5 containing 1M NaCl and re-suspended in potassium acetate buffer 50 mM, pH 5.5 containing phenylmethylsulphonyl fluoride 1 mM and sodium azide 1 mM. They were incubated overnight at 37° C for the digestion of S-glucan.

### C) Gel filtration

Aliquots of I ml from the digestion of S-glucan were repeatedly applied to a column of gel filtration (7.8 mm x 40 cm) PAK125 (Waters) packed in sodium acetate buffer 10 mM, pH 5.5 with 0.5M NaCl and eluted with the same buffer at a flow of 0.2 ml/min Fractions of 2 ml were collected and assayed to determine the concentration of protein (A₂₈₀) and for evaluation of the α-(1,3)-glucanase activity. The most active fractions were chosen. These were collected, washed and concentrated in potassium acetate buffer 100 mM, pH 5.5 in a Centricon 10 (Amicon) concentrator. The purified protein was stored at -20° C and, in these conditions, the activity of α-(1,3)-glucanase remained unaltered for at least 1 month.

Analysis using SDS-PAGE (Example 4) of samples from the digestion of S-glucan showed a majority band and other minority bands. The final purified preparation migrated as a single band in SDS-PAGE with an apparent molecular weight of approximately 72 kDa. When the molecular weight was calculated from gel filtration chromatography, the value obtained was approximately 67 kDa. The elution model for the gel filtration showed a correlation between the majority protein present in the digestions of S-glucan and the α-(1,3)-glucanase activity.

By operating in this way, it was possible to attain purification of AGN13.1 at electrophoretic homogeneity, with an 11% estimated minimum recovery. It was also observed that said enzyme is a monomeric protein.

### EXAMPLE 4

### Determination of the molecular weight

The molecular weight of the protein AGN13.1 was determined by analytical electrophoresis in denaturing conditions and by gel filtration chromatography.

### 4.1 SDS-PAGE

Electrophoresis of the AGN13.1 protein in denaturing conditions [electrophoresis on polyacrylamide gel treated with sodium dodecylsulphate, SDS-PAGE] was carried out according to the process described by Weber & Osborn [Weber & Osborn, The proteins, Vol 1, 1975, 179-221. Editor: Neurath and Hill. Academic Press, Inc., New York], using gels at 12% of acrylamide-bisacrylamide and a Mini-Protean II apparatus from Bio-Rad (US), following the manufacturer's instructions. The apparent molecular weight of the AGN13.1 protein, in the assay conditions, is approximately 72 kDa.

Alternatively, it is possible to carry out the electrophoresis of the AGN13.1 protein in denaturing conditions (SDS-PAGE) by means of the method of Laemmli (Laemmli, U.K., Nature, 1970, 227, 680-685], using gels at 4% acrylamide in the stacked gels and at 12% acrylamide in the separation gels. The samples were diluted in Laemmli buffer and boiled for 3 minutes before loading. After electrophoresis, the gels were stained with the Coomassie R-250 bright blue. In order to determine the molecular weight protein standards were used of low molecular weight proteins (Bio-Rad). The apparent molecular weight of the AGN13.1 protein, in the trial conditions is approximately 72 kDa.

### 4.2 Gel filtration chromatography

The molecular weight of the native AGN13.1 protein was determined by means of the gel filtration chromatography technique in a Sephacryl S-200 HR (Pharmacia) column (1.6 x 40 cm) calibrated with marker proteins. A value of approximately 67 kDa was obtained.

Alternatively, the molecular weight of AGN13.1 was determined from the digestion of S-glucan, by means of gel filtration chromatography in a PAK125 column (Waters) (7.8 mm x 40 cm) packed in potassium acetate buffer 100 mM, pH 5.5 containing 0.5 M NaCI and eluted with the same buffer at a flow of 0.2 ml/min, to give a value of 67 kDa approximately.

### EXAMPLE 5

### Determination of the isoelectric point

### 5.1 Isoelectric focussing

To determine the isoelectric point (IP) of the protein AGN13.1 from *T*. *harzianum* by means of isoelectric focussing, the process described by Robertson et al. [Anal. Biochem., 1987, 167, 290-294] was followed. The proteins were visualised by means of staining with Coomassie Blue. The apparent value of the IP for the AGN13.1 protein was calculated by reference to conventional marker proteins with IP values comprised between 3.5 and 9.3 (Amersham-Pharmacia). The value of apparent IP calculated by means of isoelectric focussing was approximately 7.

### 5.2 Basic analytic chromatofocussing

The IP of AGN13.1 from *T. harzianum* was determined by means of basic analytic chromatofocussing using a Polybuffer Exchanger PBE94 column (Amersham-Pharmacia) following exactly the procedure described by De la Cruz et al. [De la Cruz et al., J. Bacteriol., 1995, 177, 1864-7]. The value of apparent IP calculated by means of basic analytic chromatofocussing was approximately 7.6.

The results obtained indicate that the enzyme AGN13.1 has an IP near to neutrality.

### EXAMPLE 6

### Determination of the Michaelis-Menten constant

The Michaelis-Menten constant was determined from Lineweaver-Burk representations using the data obtained measuring the initial rate of hydrolysis of S-glucan (used as a substrate) over a range of concentrations of S-glucan ranging from 0.5 to 20 mg/ml. The values obtained were as follows:
- Km: 2.5 mg/ml; and
- Vmax: 43 µmoles of product/min.mg of protein.

### EXAMPLE 7

### Determination of the optimum temperature and deactivation temperature

The optimum temperature was determined by means of a conventional assay within a range of temperatures comprised between 20°C and 80°C. The optimum temperature for AGN13.1 was calculated at 55°C at a pH of 5.5.

The thermal stability was determined by incubating the protein AGN 13.1 purified for 30 minutes at temperatures between 20°C and 80°C, in a buffer of potassium acetate 50 mM, pH 5.5, and then measuring the remaining activity at 37° C by adding S-glucan as a substrate. The temperature of deactivation was defined as the temperature at which the specific activity was reduced by a 50%, under the conditions described earlier. The temperature of deactivation found was 50°C. These results seem to suggest that S-glucan stabilises the protein AGN13.1.

### EXAMPLE 8

### Substrate specificity and analysis of the products of reaction of AGN13.1

### 8.1 Substrate specificity

The activity of AGN13.1 from *T*. *harzianum* was assayed on polymers with bonds of α- and β-glycosides that are mentioned in Table 2, at a concentration of 5 mg/ml.

The activity of α-(1,3)-glucanase was determined by means of any of the protocols described in Example 2. The chitinase activity was determined following the protocol described by De la Cruz et al. [De la Cruz et al., Eur. J. Biochem., 1992, 206, 856-867], the protease activity by means of the protocol described by Schwartz & Barrett [Schwartz, W. & Barrett, A.J., Biochem. J., 1980, 191, 487-497], and the lytic activity by means of the protocol described by De la Cruz et al. [De la Cruz et al., Arch. Microbiol., 1993, 159, 316-322].

The results obtained are shown in Table 2, where it can be seen that the maximum activity was detected against S-glucan [a linear α-(1,3)-glucan] as substrate but no activity was observed against nigeran, a polyglucan that contains α-(1,3) and α-(1,4) alternating bonds or against other α- or β-glucans, chitin or proteins. These results show that the enzyme AGN13.1 is a specific α-(1,3)-glucanase.

**Table 2**

| **Substrate specificity of AGN13.1 purified from *T*. *haizianum*** | | |
|---|---|---|
| Substrate¹ | Type of main bond (monomer)² | α-(1,3)-glucanase³ activity (%) |
| S-glucan *(A. Niger)* | α-(1,3) (Glc) | 100 |
| IO4-S-gluca | α-(1,3) (Glc) | 0 |
| Nigeran | α-(1,3): α-(1,4) (Glc) | 0 |
| Soluble starch | α-(1,4): α-(1,6) (Glc) | 0 |
| Dextran | α-(1,6) (Glc) | 0 |
| Pachiman | β-(1,3) (Glc) | 0 |
| Glucan (S. *Cerevisiae)* | β-(1,3): β-(1,6) (Glc) | 0 |
| Laminarin | β-(1,3): β-(1,6)(Glc) | <1 |
| Carboxymethyl-cellulose | β-(1,4) (Glc) | 0 |
| Pustulan | β-(1,6) (Glc) | 0 |
| Colloidal chitin | β-(1,4) (GlcNAc) | 0 |
| Azocasein | - | 0 |
| Cell walls of *P*. *Aurantogriseum* | β-glucan:chitin | 83 |
| Cell walls of *A*. *Niger* | β-glucan:chitin | 46 |
| Cell walls *of B. Cinerea* | β-glucan:chitin | 32 |
| Cell walls of *F*.*Oxysporum* | β-glucan:chitin | 25 |
| Cell walls of *S*. *Pombe* | β-glucan | 0 |
| Cell walls of *S*. *Cerevisiae* | β-glucan | 0 |
| Cell walls of *P*. *Syringae* | β-glucan: cellulose | 0 |

| | | |
|---|---|---|
| ¹All the substrates were used at a final concentration of 4 mg/ml. | | |
| ²α-glucans have been described in detail as components of the matrix of at least the cell walls of *Penicillium* sp. and *A. niger.* | | |
| ³100% activity corresponds to 0.9 U/mg of protein. | | |

The pustulan (from *Umbilicaria papullosa)* and the pachiman (from *Poria cocos)* were from Calbiochem (USA). The azocasein, carboxymethylcellulose, chitin (from crab shell), dextran (from *Leuconostoc mesenteroids),* glucose, laminarin (from *Laminaria digitata),* nigeran (from *A. nidulans)* and soluble starch were bought from Sigma Chemical Co. (USA).

The cell walls were prepared according to the process described by Fleet & Phaff [Fleet, G.H. & Phaff, H.J., J. Biol. Chem., 1974, 249, 1717-1728]. The β-glucan from *S*. *cerevisiae* was prepared from baker's yeast, according to the protocol described by Rombous & Phaff [Rombous, F.M. & Phaff, H.J., Eur. J. Biochem., 1976, 63, 109-120]. The S-glucan of *A. niger* was prepared in accordance with known procedures [Zonneveld, Biochim. Biophys. Acta, 1971, 249, 506-514; Hasegawa, S. & Nordin, J.H., J. Biol. Chem. 1969, 244, 5460-5470]; briefly, *A. niger* 2574 was cultured in 10L flasks in 8 L of medium containing glucose (46 g/l), tartaric acid (2.66 g/l), ammonium nitrate (2.66 g/l), ammonium sulphate (0.167 g/l), potassium bicarbonate (0.932 g/l), magnesium carbonate (0.266 g/l), diacidic monoammonium phosphate (0.5 g/l), ferrous sulphate (0.0466 g/l) and zinc sulphate (0.0466 g/l). The culture was kept for a week with aeration at 28° C. Then, the culture was sterilised in an autoclave and the mycelium collected by filtration through Whartman filter paper no. 1. It was then extensively washed with 2% magnesium chloride and distilled water. All the biomass recovered, mainly material from cell walls, was lyophilised and ground in a mortar with liquid nitrogen. Next, it was treated with 5% KOH for 18 hours at 37° C. The supernatant, which mainly contained a fraction of the cell wall of alkali-soluble glucan (S-glucan), was obtained by means of centrifuging at 9000 g for 10 minutes. This fraction was precipitated by lowering the pH with glacial acetic acid at pH 7.0 and washing extensively with distilled water. The composition of the S-glucan was determined by means of infrared spectroscopy [Bacon et al., Biochim. Biophys. Acta, 1968, 158, 313-315] giving rise to a polymer that had practically pure α-(1,3) bonds.

### 8.2 Mechanism of action

The mechanism of action of AGN13.1 of *T*. *harzianum* against S-glucan was examined by measuring the production of glucose against the production of reducing sugar at different times, comparing the activities of the purified enzyme against S-glucan and S-glucan oxidised with periodate (IO4-S-glucan) and finally analysing the corresponding products of hydrolysis by means of high resolution liquid chromatography (HPLC).

Briefly, the hydrolysis of the substrates by AGN13.1 purified from *T*. *harzianum* was determined by means of HPLC using Aminex columns HPX 42-A (Bio-Rad) kept at 40° C, using water as an eluent at a flow of 0.4 ml/min. The products were detected on the basis of the refraction index and were identified by comparison with glucose standards and oligosaccharides of 2 to 4 glucose molecules.

The incubations of S-glucan with the AGN13.1 enzyme gave as a result a correlation between the reducing sugars and the glucose produced, which indicates that the glucose is responsible for almost all the reducing power and is the only product of the enzyme. On the other hand, purified AGN13.1 was unable to hydrolyse IO4-S-glucan (see Table 2), which shows that the exolytic mechanism of said enzyme, as the oxidation of the reducing terminus of the α-(1,3)-glucan impedes the activity of the AGN13.1 enzyme.

After analysis by HPLC, an initial release of glucose was detected. At longer times, the glucose peak increased, this being the final product of the hydrolysis. These results, taken as a whole, indicate a mechanism of action of the exo type for the purified AGN13.1.

This mechanism would allow the combination of AGN13.1 from *T*. *harzianum* with other lytic enzymes of the cell walls with mechanisms of action of the exo- and/or endo-hydrolytic type, with the aim of a more effective degradation of the cell wall polymers that contain α-(1,3)-glucan, along with another or other polymers, as well as having a greater enzymatic potential with anti-fungal activity against harmful fungi.

### EXAMPLE 9

### Binding of the AGN13.1 protein from T. harzianum to cell wall components of fungi.

The cell wall of the fungi is a very complex structure composed of polymers of very different types. Therefore, knowing exactly to which of the different elements that form the wall the protein binds may be very useful information for determining by means of which activity (or activities) said protein produces its anti-fungal activity.

A fractioning was carried out of the different components of the cell walls of fungi (chitin, manans, β-glucans, α-glucans, etc.) following protocols already described [Benitez et al, Arch. Microbiol., 1976, 108, 183-188]. Subsequently, binding assays were carried out for the AGN13.1 protein with each one of the purified components, following the procedure used for the purification of the AGN13.1 protein by adsorption to the cell walls of *Penicillium* sp. [see Example 3.1 Method A]. The result obtained was that the protein AGN13.1 showed a strong affinity for the fraction enriched with α-(1,3)-glucans.

### EXAMPLE 10

### Assay of the anti-fungal activity

In order to determine the anti-fungal activity of AGN13.1 of *T. harzianum,* an assay was carried out consisting, briefly, of growing in a microtitre plate well 3,000 spores of *Penicillium* sp. in PDA (Difco) diluted three times with respect to the concentration recommended by the manufacturer, along with the protein whose anti-fungal activity to be assayed for (AGN13.1), at different concentrations, keeping it at 25° C for a period of time ranging between 15 and 60 hours, and, subsequently, observing with an optical microscope. As a negative control, an assay is carried out with no protein, with PDA, spores and a protein without anti-fungal properties, for example, bovine seroalbumin (BSA). Both the inhibition of the germination of spores and the reduction in the size of the hyphae of *Penicillium* sp were carried out.

A quantification, in these conditions, of the percentage of germination of the samples treated with 150 ppm of the AGN13.1 protein of *T*. *harzianum* with respect to the control treatments showed that in the first case (spores treated with AGN13.1) the germination had reduced by almost 50%. In addition, the results show that the amount of final biomass is very much less in the samples treated with the AGN13.1 protein than in the controls.

Figure 1A shows the anti-fungal effect, determined as the inhibition of the growth of the phytopathogenic fungus *Penicillium digitatum,* produced by the protein AGN13.1, at a concentration of 225 µg/ml of BSA, on 3,000 spores of *P*. *digitatum* after 17 hours of incubation. Figure 1B shows the result of a negative control consisting of incubating for 17 hours 3,000 spores of *P*. *digitatum* with 225 µg/ml of BSA. Figure 1 shows the reduction in germination of the spores of the fungus due to the action of the protein AGN13.1, which leads to an inhibition of the growth of the fungus.

Figure 2A shows the anti-fungal effect, determined as the appearance of morphological alterations of the mycelium of the phytopathogenic fungus *P*. *digitatum,* produced by the protein AGN13.1 *from T. harzianum,* at a concentration of 225 µg/ml of BSA, on 3,000 spores of *P*. *digitatum* after 60 hours of incubation. Figure 2B shows the result of a negative control consisting of incubating for 60 hours 3,000 spores of *P*. *digitatus* with 225 µg/ml of BSA. Figure 2 shows the appearance of morphological alterations in the hyphae of the fungus *P*. *digitatum* due to the action of the protein AGN13.1 from *T. harzianum.*

### EXAMPLE 11

### Binding of the protein AGN13.1 from T. harzianum to cell walls of fungi

In order to determine the possible role of purified AGN13.1 in the antagonism of *T*. *harzianum,* said protein was evaluated in assays of binding to cell walls of fungi and of degradation of activity of said walls.

A first assay was carried out with spores of *A*. *niger* CECT 2574 in NUNC microtitre plates, in a total volume of 15 µl containing 1 µl of culture broth-potato-dextrose concentrated five-fold (5 x 24 g/l), 2 µl of a dilution of spores (150 spores) of each fungi and completing with the enzyme AGN13.1 diluted in water (previously dialysed against itself). Different concentrations of enzyme (90, 180 and 270 µg/ml) were assayed and each one of those was repeated in triplicate using spores with water as a control. The results obtained are shown in Figures 3 and 4, where the values that they represent correspond to the mean of three repetitions in each case with a determination of the error for each one of them. In Figure 5, the inhibition in the growth of the mycelium of *A*. *niger* CECT 2574 can be seen after 23 hours, in the assay concentrations, which shows the anti-fungal effect of AGN13.1 from *T*. *harzianum.*

Following the process described previously, other fungal cell walls were assayed, specifically, those of *Botrytis cinerea, Penicillium aurantogriseum, Rhizotocnia salami, Phytophtora syringae, Gibberella fujikuroi* and *Saccharomyces cerevisiae.* The results obtained showed that the enzyme AGN13.1 binds to the cell walls of the fungi *B cinerea, P. aurantogriseum* and *R. solani,* but not to those of *Phytophtora syringae, Gibberella fujikuroi* or *Saccharomyces cerevisiae.*

The enzyme Agn13.1 is very active against the cell walls of *A. niger, B. cinerea* and *P*. *aurantogriseum,* but it is not active against the cell walls of *S*. *pombe, S. cerevisiae or P. syringae* [see Table 2]. In addition, when the mechanism of action of AGN13.1 was assayed against the cell walls of *A*. *niger* or *P*. *aurantogriseum,* an exo- type mechanism was detected, in which the glucose was the majority final product of the hydrolysis. Therefore, it seems that the enzyme AGN13.1 acts as a lytic enzyme against the cell walls of phytopathogenic fungi containing polymers with α-(1,3)-glycoside bonds.

### EXAMPLE 12

### Cloning of a DNA sequence that codes for the AGN13.1 protein

In order to obtain the complete clone of cDNA of the protein AGN13.1 from *T. harzianum,* a strategy was followed that comprised the use of PCR For this, oligonucleotides were designed degenerated by microsequencing of the amino terminus and tryptic peptides from the AGN13.1 protein.

For the design of the direct oligonucleotides, sequences were used from the amino terminus and/or internal peptides of the AGN13.1 protein while for the design of reverse oligonucleotides, sequences were used from the vector in which the gene library of cDNA was constructed.

With said oligonucleotides and under the experimental conditions that are described below [see Example 12.5] some specific bands were obtained of the sequence coding for the protein of the invention which was subcloned in the commercial vector pGEM-T (Promega, WI, USA). The sequencing of the amplified bands confirmed that these were the desired clone fragments. Next, the fragments obtained by PCR were marked with radioisotopes and used as a probe for examining a library of cDNA from *T harziamim* CECT 2413.

From all the clones examined, some positive clones were obtained whose analysis showed that they carried some inserts that completely encompassed the ORF and some of the neighbouring sequences of the promoter and terminator region.

### 12.1 Extraction of gDNA

The gDNA from *Trichoderma harzianum* CECT 2413 was obtained following the protocol described by Kaiser et al. (1994) [Methods in yeast genetics. A Cold Spring Harbour Laboratory Press, Cold Spring Harbour, New York] with some modifications including the addition of a purification step with phenol. Briefly, 0.3-0.5 g of lyophilised mycelium are re-suspended in Tris-HCl 50 mM and EDTA 20 mM and homogenised. 200 ml of 10% sodium dodecylsulphate (SDS) are added. Next, the mixture was incubated at 65° C for 30 minutes, the sodium acetate added and the incubation performed for a further 30 minutes at 4° C. Finally, the mixture was centrifuged and the supernatant treated with phenol and precipitated with ethanol.

### 12.2 Extraction of total DNA

The total DNA from *T*. *harzianum* CECT 2413 was extracted using the method of phenol acid described by Chomczynski & Sacchi [Chomczynski & Sacchi, Anal. Biochem., 1987, 162, 156] with slight modifications.

### 12.3 Construction of a cDNA gene library

From the total DNA of *T*. *harzicmum* CECT 2413, obtained after cultivating said fungi in MM with cell walls of *Botrytis* at 0.5% as a source of carbon for 9 hours, the mRNA was isolated. For this, oligo(dT)cellulose affinity chromatography (Stratagene, La Jolla, CA) was used. Next, the cDNA was synthesised using the commercial kit "ZAP-cDNA synthesis kit" (Stratagene). To the cDNA so obtained, the *Eco* RI and *Xho* I adapters were added and it was bound to the vector Uni-Zap XR (Stratagene, La Jolla, CA). Finally, to package the cDNA found to the λ phage the packing kit "Gigapack III gold packaging extract" (Strategene, La Jolla, CA) was used following the manufacturer's instructions.

### 12.4 Microsequencing of proteins

In order to determine the amino terminus of the AGN13.1 protein, and to obtain internal fragments that would allow the cloning of the corresponding gene, a microsequencing was carried out following the method of Edmans Matsudaira [A practical guide to protein and peptide purification for microsequencing. Academic Press, Inc., New York, Editor; Edmans Matsudaira 1989]. The results obtained were as follows:

Amino terminus: ASSADRLVFSHFMIGIVGD [SEQ. ID No. 4]
Internal peptide: DDTVYVAALLKTAGSVTVTSGGT [SEQ. ID No. 5]

### 12.5 Polymerase chain reaction (PCR)

In the PCR experiments, as a sense oligonucleotide, a degenerated oligonucleotide was used designed from the internal peptide of the AGN13.1 protein and as anti-sense oligonucleotide a sequence corresponding to the plasmid in which the gene library is constructed (pBluescript II, Strategene, La Jolla, CA). Specifically, the direct and reverse oligonucleotides were as follows: 
Direct: 5'-GATGATACNGTYTAYGTNGCNGC-3' [SEQ. ID No. 6]
Reverse: 5'-GTAATACGACTCACTATAGGGC-3' [SEQ. ID No. 7]
 where N is inosine, and Y is T or C.

To carry out the PCR the following parameters were used:
- 1 cycle of denaturing at 95° C for 1 minute, followed by
- 35 cycles of synthesis comprising:
   - hybridisation phase at 55° C for 1 minute,
   - an elongation phase at 72° C for 1 minute, and
   - a denaturing phase at 95° C for 1 minute; and finally
- 1 additional cycle of elongation of 7 minutes at 72° C to complete the unfinished products

In all cases, the PCR was carried out using cDNA from the cleavage of the gene library as a mould and using 100 pmoles of each oligonucleotide in 25 µl of total volume of PCR.

### 12.6 Examination of the cDNA library

The examination of the cDNA gene library was carried out following conventional methods described by Sambrook et al. [Molecular Cloning. A Laboratory Manual, Cold Spring Harbour, NY, 1989], using probes labelled radioactively with α³²P-dCTP following the random seeding method described by Feingerg and Volgelstein (1983) [Anal. Biochem, 132:6]. For this, the commercial kit "Oligolabelling kit" (Pharmacia, Sweden) was used following the manufacturer's instructions.

### 12.7 Cloning of cDNA

For the design of the direct oligonucleotide, the sequence of the amino terminus of AGN13.1 was used, and for the design of the reverse oligonucleotide a sequence of the plasmid was used in which the gene library was constructed [SEQ. ID. No. 7].

With these oligonucleotides and under the experimental conditions described previously [see Example 8.5] a specific band of 900 nucleotides was obtained which was subcloned into the commercial vector pGEM-T (Promega, WI). Subsequently, sequencing of this band confirmed that this was a fragment of the desired clone. Next, the fragment obtained by PCR is labelled radioactively and used as a probe for examining a gene library of λ, Zap constructed from RNA extracted from *T*. *harzianum* CECT 2413 after 48 hours of induction in MM with cell walls of *Botrytis.* From 50,000 clones examined several clones were obtained, of which only the largest (2.2 kb) were subcloned. For the analysis of the clone, in the first place, the phage was cleaved following the manufacturer's instructions, then proceeding to the sequencing of both chains of the insert that carried it. In said clone, all the ORF was found along with fragments of the non-coding 5' and 3' regions.

### EXAMPLE 13

### Molecular cloning of cDNA to the agn13.1 gene.

The molecular cloning of the *agn13.1* gene that codes for the AGN13.1 from *T. harzianum* was carried out by the techniques of PCR based on the information obtained from the amino terminus of the sequence of amino acids of said protein [Example 12].

Briefly, a cDNA gene library was constructed in a Uni-Zap XR vector (Stratagene) from the tail poli(A)+ of RNA isolated from *T*. *harzianum* CECT 2413 grown in conditions that simulated antagonism, which, in this case where minimum medium containing 0.5% of cell walls of *B*. *cynerea* or of *P*. *syringae.* The cDNA was synthesised using the commercial kit "ZAP-cDNA synthesis kit" (Stratagene) over every batch of poli(A)+ of RNA, the Uni-Zap vector (Stratagene) was bound and packaged in "Gigapack III gold packaging extract" (Stratagene), following the manufacturer's instructions. The concentration of the primary gene library was approximately 2x10⁶ independent plate forming units (PFU).

The total DNA was isolated from said gene library and used as a template for the PCR using as direct initiator (sense) the oligonucleotide whose sequence is shown in the SEQ. ID. No. 6 and as anti-sense oligonucleotide a commercial initiator of T3 [SEQ. ID. No. 8]. The amplification of the DNA was performed in the following conditions: 95°C for 1 minute; 55°C for 1 minute, and 72°C for 1 minute, repeating this cycle 35 times. A single amplified fragment was obtained of 1,800 pb which was subcloned in the plasmid pGEM-T (Promega) and sequenced. The fragment of amplified cDNA corresponds to the region of the carboxyl terminus of the gene *agn13.1.* Therefore, to obtain a cDNA of said gene, of full length, said fragment amplified by PCR, labelled with ³²P, was used as a probe to examine the gene library of cDNA ZAPII previously obtained. The hybridisations were carried out by means of a conventional protocol [Lora et al., Mol. Gen. Genet., 1995, 247, 639-645]. After examination of approximately 40,000 PFU of said gene library of cDNA various positive clones were obtained. Of these, the one that contained the biggest insert [2,190 pb] was selected for its subsequent characterisation. Said insert was subcloned into the plasmid pGEM-T (Promega) and sequenced. The insert contained a single ORF of 1908 pb, a 5' sequence not translated of 110 pb and a 3' sequence not translated of 133 bp. The translation of the product of the *agn13.1* gene has a sequence of 636 amino acids with an estimated molecular weight of approximately 67 kDa. Comparison of the sequence deduced with the sequence of the amino terminus of AGN13.1 determined experimentally indicates that the mature protein begins at the position 38. Therefore, the α-(1,3)-glucanase of *T*. *harzianum* contains 597 groups of amino acid and has an estimated molecular weight of 63,789 kDa and an estimated IP of 5.36. The predicted protein contains the peptides sequenced from the purified protein, which shows that the cDNA of cloned *agn13*.*1* codes for the purified AGN13.1.

### EXAMPLE 14

### Model for expression of mRNA of agn13.1 and the protein AGN13.1

Northern Blot and Western Blot analyses were carried out to establish whether the induction of *agn13.1* occurred preferably under similar conditions to those that implicate the response of antagonism of *T*. *harzianum.*

### 14.1 Northern Blot

This analysis was carried out by means of a conventional protocol. The results obtained show that the mRNA of AGN13.1 was present in the mycelia grown in chitin and in fungal cell walls. The levels of these mRNA were different in the conditions in which the signal appeared after 9 hours of induction (cultures with chitin and fungal cell walls) while they were similar after 48 hours.

The relatively low presence of mRNA of AGN 13.1 in *T. harzianum* grown with walls of *Aspergillus* after 9 hours may be due to a higher release of glucose from these cell walls or to a lower efficiency of a possible inductor.

Therefore, it seems that the mRNA of AGN13.1 is produced in conditions of carbon deficiency over a short period of induction except in a medium with glucose at 0.1% where no signal is observed, probably as a result of the presence of glucose in sufficient quantities for catabolic repression. At longer times, the catabolic de-repression is a general situation for *T*. *harzianum* cultured in conditions of carbon deficiency. Therefore, similar levels of mRNA of AGN13.1 are produced, although certain cell wall inductors may exist.

With regards to the nitrogen conditions, no signal was observed, which suggests that the transcription of mRNA of ADN13.1 depends only on the carbon source.

### 14.2 Western blot

This analysis was carried out following general specifications [Ausubel et al., Analysis of proteins, Vol. 2, John Wiley & Sons, Inc. N. York, 1994] and using a commercial system of protein transfer (Amersham-Pharmacia). Briefly, 20 µg of AGN13.1 protein were transferred from the polyacrylamide gel to a nitrocellulose membrane at 400 V for 2 hours. Then, the membrane was blocked with powdered milk at 5% in TBS and incubated for 12 hours with anti-AGN13.1 obtained from rabbit. It was then washed with Tween® at 0.05% in TBS and incubated with an anti-rabbit anti-body conjugated with horseradish peroxidase (Sigma Chemical Co.). Then, the membrane was washed again with Tween® at 0.05% and finally developed in the presence of protein with 2,5-chloronaphtol and hydrogen peroxide.

To carry out this assay, *T*. *harzianum* was grown in the presence of the following carbon sources: 2% glucose, 0.1% glucose, 1.5% chitin, cell walls of *P*. *syringae* 0.5%, cell walls of *P*. *aurantogriseum* 0.5% and cell walls of *A*. *niger* 0.5% and in conditions of nitrogen deficiency.

The results obtained show that the extracellular production of AGN13.1 of *T*. *harzianum* in the presence of different sources of carbon is conditioned by the absence of a source of assimilable carbon, above all by the presence of cell walls or polysaccharides, such as chitin, in the culture media. In media of 2% glucose, with or without nitrogen limitation, the protein is not produced.

In accordance with these results, the catabolic de-repression is basically what leads to the production of the protein AGN13.1.

LIST OF SEQUENCES

(1) GENERAL INFORMATION:
(i) APPLICANT:
(A) NAME: NEWBIOTECHNIC, S.A.
(B) STREET: Avda. Américo Vespucio, 69
(C) CITY: Sevilla
(D) PROVINCE: Sevilla
(E) COUNTRY: Spain
(F) POSTAL CODE (ZIP): 41092
(G) TELEPHONE: 954 081 031
(H) FAX: 954 081 034
(i) APPLICANT:
(A) NAME: UNIVERSIDAD DE SEVILLA
(B) STREET: Valparaíso, 5
(C) CITY: Sevilla
(D) PROVINCE: Sevilla
(E) COUNTRY: Spain
(F) POSTAL CODE (ZIP): 41013
(G) TELEPHONE: 954 237 871
(H) FAX: 954 238 537

(ii) TITLE OF THE INVENTION:

ANTIFUNGIC COMPOSITIONS AND METHOD FOR CONTROLLING FUNGI

(iii) NUMBER OF SEQUENCES: 8

(iv) COMPUTER READABLE FORM:
(A) TYPE OF SUPPORT: Floppy disk
(B) COMPUTER: IBM PC compatible
(C) OPERATIVE SYSTEM: PC-DOS/MS-DOS
(D) SOFTWARE: PatentIn Release #1.0, Version #1.30
(OEP)

(2) INFORMATION OF SEQ. ID. No. 1:

(i) SEQUENCE CHARACTERISTICS:
(A) LENGTH: 636 amino acids
(B) TYPE: amino acid
(C) STRANDNESS: unknown
(D) TOPOLOGY: unknown

(ii) TYPE OF MOLECULE: Protein

(xi) DESCRIPTION OF SEQUENCE SEQ. ID. No. 1:
Met Leu Gly Val Phe Arg Arg Leu Arg Leu Gly Ala Leu Ala Ala Ala
1               5                   10                  15
Ala Leu Ser Ser Leu Gly Ser Ala Ala Pro Ala Asn Val Ala Ile Arg
            20                  25                  30
Ser Leu Glu Glu Arg Ala Ser Ser Ala Asp Arg Leu Val Phe Cys His
        35                  40                  45
Phe Met Ile Gly Ile Val Gly Asp Arg Gly Ser Ser Ala Asp Tyr Asp
    50                  55                  60
Asp Asp Met Gln Arg Ala Lys Ala Ala Gly Ile Asp Ala Phe Ala Leu
65                  70                  75                  80
Asn Ile Gly Val Asp Gly Tyr Thr Asp Gln Gln Leu Gly Tyr Ala Tyr
                85                  90                  95
Asp Ser Ala Asp Arg Asn Gly Met Lys Val Phe Ile Ser Phe Asp Phe
            100                 105                 110
Asn Trp Trp Ser Pro Gly Asn Ala Val Gly Val Gly Gln Lys Ile Ala
        115                 120                 125
Gln Tyr Ala Asn Arg Pro Ala Gln Leu Tyr Val Asp Asn Arg Pro Phe
    130                 135                 140
Ala Ser Ser Phe Ala Gly Asp Gly Leu Asp Val Asn Ala Leu Arg Ser
145                 150                 155                 160
Ala Ala Gly Ser Asn Val Tyr Phe Val Pro Asn Phe His Pro Gly Gln
                165                 170                 175
Ser Ser Pro Ser Asn Ile Asp Gly Ala Leu Asn Trp Met Ala Trp Asp
            180                 185                 190
Asn Asp Gly Asn Asn Lys Ala Pro Lys Pro Gly Gln Thr Val Thr Val
        195                 200                 205
Ala Asp Gly Asp Asn Ala Tyr Lys Asn Trp Leu Gly Gly Lys Pro Tyr
    210                 215                 220

Leu Ala Pro Val Ser Thr Trp Val Phe Asn His Phe Gly Pro Glu Val
225                 230                 235                 240
Ser Tyr Ser Lys Asn Trp Val Phe Pro Ser Gly Pro Leu Ile Tyr Asn
                245                 250                 255
Arg Trp Gln Gln Val Leu Gln Gln Gly Phe Pro Arg Val Glu Ile Val
            260                 265                 270
Thr Trp Asn Asp Tyr Gly Glu Ser His Tyr Val Gly Pro Leu Lys Ser
        275                 280                 285
Lys Gln Phe His Asp Gly Asn Ser Lys Trp Val Asn Asp Met Pro His
    290                 295                 300
Asp Gly Phe Leu Asp Leu Ser Lys Pro Phe Ile Ala Ala Tyr Lys Asn
305                 310                 315                 320
Arg Asp Thr Asp Ile Ser Lys Tyr Val Gln Asn Glu Gln Leu Val Tyr
                325                 330                 335
Trp Tyr Arg Arg Asn Leu Lys Ala Leu Asp Cys Asp Ala Thr Asp Thr
            340                 345                 350
Thr Ser Asn Arg Pro Ala Asn Asn Gly Ser Gly Asn Tyr Phe Glu Gly
        355                 360                 365
Arg Pro Asp Gly Trp Gln Thr Met Asp Asp Thr Val Tyr Val Ala Ala
    370                 375                 380
Leu Leu Lys Thr Ala Gly Ser Val Thr Val Thr Ser Gly Gly Thr Thr
385                 390                 395                 400
Gln Thr Phe Gln Ala Asn Ala Gly Ala Asn Leu Phe Gln Ile Pro Ala
                405                 410                 415
Ser Ile Gly Gln Gln Lys Phe Ala Leu Thr Arg Asn Gly Gln Thr Val
            420                 425                 430
Phe Ser Gly Thr Ser Leu Met Asp Ile Thr Asn Val Cys Ser Cys Gly
        435                 440                 445
Ile Tyr Asn Phe Asn Pro Tyr Val Gly Thr Ile Pro Ala Gly Phe Asp
    450                 455                 460
Asp Pro Leu Gln Ala Asp Gly Leu Phe Ser Leu Thr Ile Gly Leu His
465                 470                 475                 480
Val Thr Thr Cys Gln Ala Lys Pro Ser Leu Gly Thr Asn Pro Pro Val
                485                 490                 495
Thr Ser Gly Pro Val Ser Ser Leu Pro Ala Ser Ser Thr Thr Arg Ala
            500                 505                 510

Ser Ser Pro Pro Pro Val Ser Ser Thr Arg Val Ser Ser Pro Pro Val
        515                 520                 525
Ser Ser Pro Pro Val Ser Arg Thr Ser Ser Ala Pro Pro Pro Pro Gly
    530                 535                 540
Asn Ser Thr Pro Pro Ser Gly Gln Val Cys Val Ala Gly Thr Val Ala
545                 550                 555                 560
Asp Gly Glu Ser Gly Asn Tyr Ile Gly Leu Cys Gln Phe Ser Cys Asn
                565                 570                 575
Tyr Gly Tyr Cys Pro Pro Gly Pro Cys Lys Cys Thr Ala Phe Gly Ala
            580                 585                 590
Pro Ile Ser Pro Pro Ala Ser Asn Gly Arg Asn Gly Cys Pro Leu Pro
        595                 600                 605
Gly Glu Gly Asp Gly Tyr Leu Gly Leu Cys Ser Phe Ser Cys Asn His
    610                 615                 620
Asn Tyr Cys Pro Pro Thr Ala Cys Gln Tyr Cys Glx
625                 630                 635

(2) INFORMATION OF SEQ. ID. No. 2:

(i) SEQUENCE CHARACTERISTICS:
(A) LENGTH: 2256 bases
(B) TYPE: nucleic acid
(C) STRANDNESS: single
(D) TOPOLOGY: linear

(ii) TYPE OF MOLECULE: DNA

(xi) DESCRIPTION OF SEQUENCE SEQ. ID. No. 2:
ATGTTTCCAT CTGTCGTTGT TTGCATCTGC CGGCCTCCGC GCGCGCCTGG TACCTATTTC   60
TCGGGGTTAA GTCTCTAAGA GAGTTTCATG CACAGGCAGT AGACAGGAAT ATATAAACGC  120
ACTGGTTGAG GCCTATGTTA TGCCAAGGAC TCCAAACCAA CGGTCTGCAA GGACCACAGA  180
TCCAGTCTCT CAAGCAAAAT CCATCCAAAA CACCCTCAAT GTTGGGCGTT TTCCGCCGCC  240
TCAGGCTCGG CGCCCTTGCC GCCGCAGCTC TGTCTTCTCT CGGCAGTGCC GCTCCCGCCA  300
ATGTTGCTAT TCGGTCTCTC GAGGAACGTG CTTCTTCTGC TGACCGTCTC GTATTCTGTC  360
ATTTCATGAT TGGGATCGTG GGTGACCGTG GCAGCTCGGC AGATTATGAT GACGATATGC  420
AACGTGCCAA AGCCGCTGGC ATTGACGCCT TCGCCCTGAA CATCGGCGTT GACGGCTATA  480
CCGACCAGCA GCTCGGCTAT GCCTATGACT CTGCCGATCG TAATGGCATG AAAGTCTTCA  540
TTTCATTTGA TTTCAACTGG TGGAGCCCCG GCAATGCAGT TGGTGTTGGC CAGAAGATTG  600

CGCAGTATGC CAACCGCCCT GCCCAGCTGT ATTCGACAAC CGGCCATTCG CCTCTTCCTT 660
CGCCGGTGAC GGTCTGGATG TAAATGCGTT GCGCTCTGCT GCAGGCTCCA ACGTTTACTT 720
TGTGCCCAAC TTCCACCCTG GTCAATCTTC CCCCTCCAAC ATTGATGGCG CCCTTAACTG 780
GATGGCCTGG GATAATGATG GAAACAACAA GGCACCCAAG CCGGGCCAGA CTGTCACAGT 840
GGCAGACGGT GACAACGCTT ATAAGAATTG GTTGGGTGGC AAGCCTTACC TGGCGCCTGT 900
CTCAACTTGG GTTTTCAACC ATTTCGGGCC CGAAGTTTCA TATTCCAAGA ATGGGTTTTC 960
CCAAGTGGGC CTCTGATCTA TAACCGGTGG CAACAAGTCT TGCAGCAAGG GTTCCCAAGG 1020
GTTGAGATCG TTACCTGGAA TGACTACGGG GAATCTCACT ACGTCGGTCC CCTGAAGTCT 1080
AAGCAATTTC ATGATGGGAA CTCCAAATGG GTCAATGATA TGCCCCACGA TGGATTCCTG 1140
GATCTTTCGA AGCCGTTCAT AGCCGCATAT AAAAACAGGG ATACCGACAT CTCCAAGTAT 1200
GTTCAAAATG AGCAGCTTGT TTACTGGTAC CGCCGCAACT TAAAGGCACT GGACTGTGAC 1260
GCCACCGACA CAACCTCTAA CCGCCCGGCT AACAATGGAA GCGGCAATTA CTTTGAGGGA 1320
CGCCCCGATG GTTGGCAAAC TATGGATGAT ACGGTTTACG TGGCGGCACT TCTCAAGACT 1380
GCCGGTAGCG TCACGGTCAC GTCTGGTGGC ACCACTCAAA CGTTCCAGGC CAACGCCGGA 1440
GCCAATCTCT TCCAAATCCC GGCCAGCATC GGCCAGCAAA AGTTTGCTCT GACTCGTAAC 1500
GGTCAGACCG CTTTAGCGGA ACCTCATTGA TGGATATCAC CAACGTTTGC TCTTGCGGTA 1560
TCTACAACTT CAACCCATAT GTTGGCACCA TTCCTGCCGG CTTTGACGAC CCTCTTCAGG 1620
CTGACGGTCT TTTCTCTTTG ACCATCGGAT TGCACGTCAC AACTTGTCAG GCCAAGCCAT 1680
CTCTTGGAAC TAACCCTCCT GTCACTTCCG GCCCTGTGTC CTCGCTTCCA GCTTCCTCCA 1740
CCACCCGCGC ATCCTCGCCG CCTCCTGTTT CTTCAACTCG TGTCTCTTCT CCCCCTGTCT 1800
CTTCCCCTCC AGTTTCTCGC ACCTCTTCTG CCCCTCCCCC TCCGGGCAAC AGCACGCCGC 1860
CATCGGGTCA GGTTTGCGTT GCCGGCACCG TTGCCGACGG CGAGTCTGGC AACTACATCG 1920
GCCTGTGCCA ATTCAGCTGC AACTACGGTT ACTGCCCACC AGGACCGTGT AAGTGCACCG 1980
CCTTTGGTGC TCCCATCTCG CCACCGGCAT CCAACGGCCG CAACGGCTGC CCTCTGCCGG 2040
GAGAAGGCGA TGGTTATCTG GGCCTGTGCA GTTTCAGTTG TAACCATAAT TACTGCCCGC 2100
CAACGGCATG TCAATACTGC TAGGAGGGAT CAATCTCAGT ATGAGTATAT GGAGGCTGCT 2160
GAAGGACCAA TTAGCTGTTC TTATCGGCAG ACGAAACCCA TAGAGTAAGA AGTTAAATAA 2220
AATGCAATTA ATGTGTTTTC AAAAAAAAAA AAAAAA 2256

(2) INFORMATION OF SEQ. ID. No. 3:

(i) SEQUENCE CHARACTERISTICS:
(A) LENGTH: 1046 bases
(B) TYPE: nucleic acid
(C) STRANDNESS: single
(D) TOPOLOGY: linear

(ii) TYPE OF MOLECULE: DNA

(xi) DESCRIPTION OF SEQUENCE SEQ. ID. No. 3:

TGGAGCCCCG GCAATGCAGT TGGTGTTGGC CAGAAGATTG CGCAGTATGC CAACCGCCCT 60
GCCCAGCTGT ATGTCGACAA CCGGCCATTC GCCTCTTCCT TCGCCGGTGA CGGTCTGGAT 120
GTAAATGCGT TGCGCTCTGC TGCAGGCTCC AACGTTTACT TTGTGCCCAA CTTCCACCCT 180
GGTCAATCTT CCCCCTCCAA CATTGATGGC GCCCTTAACT GGATGGCCTG GGATAATGAT 240
GGAAACAACA AGGCACCCAA GCCGGGCCAG ACTGTCACAG TGGCAGACGG TGACAACGCT 300
TATAAGAATT GGTTGGGTGG CAAGCCTTAC CTGGCGCCTG TCTCAACTTG GGTTTTCAAC 360
CATTTCGGGC CCGAAGTTTC ATATTCCAAG AACTGGGTTT TCCCAAGTGG GCCTCTGATC 420
TATAACCGGT GGCAACAAGT CTTGCAGCAA GGGTTCCCAA GGGTTGAGAT CGTTACCTGG 480
AATGACTACG GGGAATCTCA CTACGTCGGT CCCCTGAAGT CTAAGCAATT TCATGATGGG 540
AACTCCAAAT GGGTCAATGA TATGCCCCGT GGCGGCACTT CTCAAGACTG CCGGTAGCGT 600
CACGGTCACG TCTGGTGGCA CCACTCAAAC GTTCCAGGCC AACGCCGGAG CCAATCTCTT 660
CCAAATCCCG GCCAGCATCG GCCAGCAAAA GTTTGCTCTG ACTCGTAACG GTCAGACCGT 720
CTTTAGCGGA ACCTCATTGA TGGATATCAC CAACGTTTGC TCTTGCGGTA TCTACAACTT 780
CAACCCATAT GTTGGCACCA TTCCTGCCGG CTTTGACGAC CCTCTTCAGG CTGACGGTCT 840
TTTCTCTTTG ACCATCGGAT TGCACGTCAC AACTTGTCAG GCCAAGCCAT CTCTTGGAAC 900
TAACCCTCCT GTCACTTCCG GCCCTGTGTC CTCGCTTCCA GCTTCCTCCA CCACCCGCGC 960
ATCCTCGCCG CCTCCTGTTT CTTCAACTCG TGTCTCTTCT CCCCCTGTCT CTTCCCCTCC 1020
AGTTTCTCGC ACCTCTTCTC CCCTCC 1046

(2) INFORMATION OF SEQ. ID. No. 4:

(i) SEQUENCE CHARACTERISTICS:
(A) LENGTH: 19 amino acids
(B) TYPE: amino acid
(C) STRANDNESS: unknown
(D) TOPOLOGY: unknown

(ii) TYPE OF MOLECULE: Peptide

(v) TYPE OF FRAGMENT: N-terminal

(xi) DESCRIPTION OF SEQUENCE SEQ. ID. No. 4:

Ala Ser Ser Ala Asp Arg Leu Val Phe Ser His Phe Met Ile Gly Ile
1               5                   10                  15
Val Gly Asp

(2) INFORMATION OF SEQ. ID. No. 5:

(i) SEQUENCE CHARACTERISTICS:
(A) LENGTH: 23 amino acids
(B) TYPE: amino acid
(C) STRANDNESS: unknown
(D) TOPOLOGY: unknown

(ii) TYPE OF MOLECULE: Peptide

(iii) TYPE OF FRAGMENT: Internal

(xi) DESCRIPTION OF SEQUENCE SEQ. ID. No. 5:

Asp Asp Thr Val Tyr Val Ala Ala Leu Leu Lys Thr Ala Gly Ser Val
1               5                   10                  15
Thr Val Thr Ser Gly Gly Thr
            20

(2) INFORMATION OF SEQ. ID. No. 6:

(i) SEQUENCE CHARACTERISTICS:
(A) LENGTH: 23 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 6

GATGATACNG TYTAYGTNGC NGC 23
[N = Inosine]

(2) INFORMATION OF SEQ. ID. No. 7:

(i) SEQUENCE CHARACTERISTICS:

(A) LENGTH: 22 bases
(B) TYPE: nucleic acid
(C) STRANDNESS: single
(D) TOPOLOGY: linear

(ii) TYPE OF MOLECULE: Other DNA
(A) DESCRIPTION: /desc: ""synthetic"

(xi) DESCRIPTION OF SEQUENCE SEQ. ID. No. 7

GTAATACGAC TCACTATAGG GC 22

(2) INFORMATION OF SEQ. ID. No. 8:

(i) SEQUENCE CHARACTERISTICS:
(A) LENGTH: 20 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 8

AATTAACCCT CACTAAAGGG 20

## Claims

1. Use as an anti-fungal agent of a protein having, at least, α-(1,3)-glucanase enzymatic activity.

2. Use according to claim 1, in which said protein has the ability to bind to a cell wall of a fungus and inhibit the germination of spores and/or the growth of the mycelium of a fungus.

3. Use according to claim 2, in which said fungus is a fungus that contains on its cell wall a polymer having α-(1,3)-glycoside bonds.

4. Use according to claim 2, in which said fungus is a fungus containing S-glucan in its cell wall.

5. Use according to claim 1, in which said protein has a sequence of amino acids selected from:
a) the sequence of amino acids shown in SEQ. ID. No. 1, and
b) a sequence of amino acids substantially homologous and functionally equivalent to the sequence of amino acids shown in SEQ. ID. No. 1.

6. Use according to claim 1, in which said protein is a α-(1,3)-glucanase from *T. harzianum.*

7. Use according to claim 6, in which said α-(1,3)-glucanase from *T*. *harzianum* is the protein identified as AGN13.1 and has the sequence of amino acids shown in the SEQ. ID. No. 1.

8. Use according to claim 2, in which said fungus is a fungus that contains in its cell wall a polymer having α-(1,3)-glycoside bonds, selected from a pathogenic fungus of plants, a pathogenic fungus of animals, a pathogenic fungus of humans and a fungus that contaminates foodstuffs or industrial, agricultural or livestock installations.

9. An anti-fungal composition that comprises, at least, a protein having, at least, α-(1,3)-glucanase activity, along with an inert vehicle.

10. A composition for controlling phytopathogenic fungi that comprises an effective anti-fungal quantity of, at least, a protein having, at least, α-(1,3)-glucanase activity, along with, optionally, one or more agriculturally acceptable vehicles.

11. A pharmaceutical composition for preventing and/or treating pathogenic fungi of animals, including pathogens of humans, that comprises an effective anti-fungal quantity of, at least, a protein having, at least, α-(1,3)-glucanase activity, along with, optionally, one or more pharmaceutically acceptable excipients.

12. A composition according to any of claims 9 to 11, in which said protein has the ability to bind to a cell wall of a fungus and inhibit the germination of spores and/or the growth of the mycelium of the fungus.

13. A composition according to claim 12, in which said fungus is a fungus that contains in its cell wall a polymer having α-(1,3)-glycoside bonds.

14. A composition according to any of claims 9 to 11, in which said protein has a sequence of amino acids selected from:
a) the sequence of amino acids shown in SEQ. ID. No. 1, and
b) a sequence of amino acids substantially homologous and functionally equivalent to the sequence of amino acids shown in SEQ. ID. No. 1.

15. A composition according to any of claims 9 to 11, in which said protein is a α(1,3)-glucanase from *T*. *harzianum.*

16. A composition according to claim 15, in which said α-(1,3)-glucanase from *T*. *harzianum* is the protein identified as AGN13.1 and has the sequence of amino acids shown in SEQ. ID. No. 1.

17. A composition according to claim 9, which further comprises one or more fungicides selected from natural, recombinant or synthetic fungicides.

18. A composition according to any of claims 10 or 11, which further comprises one or more proteins, with equal or different enzymatic activities for the modification or degradation of fungal cell walls.

19. A composition according to claim 18, which comprises one or more lytic enzymes able to modify or degrade cell walls of fungi.

20. A composition according to claim 18, which further comprises one or more chemical fungicides.

21. A method for controlling a phytopathogenic fungus in a plant, which comprises the stage of applying to said plant, or to the medium that surrounds it, an effective quantity of a composition for controlling said phytopathogenic fungus according to claim 10.

22. A method according to claim 21, in which said composition is applied to the parts of the plant above ground.

23. A method according to claim 21, in which said composition is applied to the fruit, before or after harvest.

24. A method for de-infesting, preventing and/or treating the infection caused by pathogenic fungi of animals in livestock facilities which comprises the stage of applying to said livestock facilities an effective quantity of said pharmaceutical composition according to claim 11.

25. A method for controlling the fungal contamination in assay samples for analysis which comprises bringing into contact said assay sample and an effective quantity of said composition according to claim 9.
